# EUROPEAN PATENT APPLICATION

(11) **EP 1 396 230 A1**
(43) Date of publication of application: **10.03.2004**
(21) Application number: 03020293.1
(22) Date of filing: 08.09.2003
(51) Int. Cl.: A61B 10/00

(54) **Improved needle instrument for taking osteomedullary bioptical samples**

(30) Priority: 09.09.2002 IT bo20020083
(71) Applicant: Avaltroni, Paolo, 46100 Mantova (IT)
(72) Inventor: Avaltroni, Paolo, 46100 Mantova (IT)
(74) Representative: Dall'Olio, Giancarlo

(57) **Abstract**

The needle instrument (100) for performing biopsies of bone or osteo-medullary tissue comprises a hollow cannula (1), having a sharpened distal end (1a) for penetrate in a patient's osteo-medullary tissue and for receiving a sample (10) of said tissue into a hollow distal portion (3) of said cannula.

The needle instrument (100) moreover comprises a pointed stylet (4), slidably insertable inside the inner cannula (5).

Inside the cannula (1) there is fixed a cylindrical inner cannula (5), which extends at least for a part of said cannula (1) comprising the hollow distal portion (3). The inner cannula (5) is provided with a pair of longitudinal, counter-facing protrusions (51), which are the continuation of corresponding cylindrical sectors of the same inner cannula (5) and that extend, in the aforesaid hollow distal portion (3), towards the distal end of said cannula (1).

As the sample (10) is constrained to rotate together with the cannulas (1,5), its distal end, which is still connected to the surrounding tissue after pushing down the needle instrument into the bone, is subjected to torsional forces which cause its separation from said tissue.

## Description

The present invention fits into the technical sector concerning the production of surgical instruments which have to be used for taking tissue samples from a living organism, in order to make a diagnosis of some pathologies that can eventually affect said organism.

More particularly, the present invention relates to an improved needle instrument for performing a biopsy of an osteo-medullary tissue.

So far, several kinds of instruments for performing biopsies have been used, both for taking samples of rigid tissues, such as bone tissue or osteo-medullary tissues, and soft tissues.

More particularly, as far as it regards the rigid tissue biopsies, needle instruments comprising a cannula provided with a handle at its proximal end are known; the cannula distal portion has a tapered, sharp end.

Inside the cannula is slidably inserted a stylet, comprising a cylindrical needle provided with a handle at its proximal end, the distal end thereof being suitably pointed.

The stylet is made so as its point slightly protrudes from the cannula distal end, once the stylet is fully inserted thereinto.

The above described instrument is used by manually inserting the cannula, having the stylet inserted in place, into the rigid tissue to be sampled, through the skin and other fat or muscular tissues interposed thereto.

The stylet point is shaped to allow the biopsy instrument to perforate the body tissues, comprising the hard, external layer of the bone tissue. Moreover, it prevents undesired tissue bits, not belonging to the sampling area, from entering into the cannula.

After the instrument distal end has reached the sampling area, the stylet is pulled out from the cannula by sliding it back from the distal end. The cannula is then pushed and rotated, so as to separate a cylindrical tissue portion, which is technically known as "frustule", and to retain it inside the cannula.

Subsequently, the cannula is rotated and swinged again, in order to obtain the complete detaching of said cylindrical portion from the surrounding tissue, and to separate the frustule therefrom.

The cannula is then pushed out from the patient's body, together with the associated frustule, which has an approximately cylindrical shape.

The biopsy instrument of the above described kind has a series of heavy drawbacks, due to a great complexity of the sampling operation, and also because the same operation are considerably invasive.

Indeed, there is a high probability that the biopsic sample, even after the aforesaid operation has been performed, would not be totally separated from the surrounding tissue, and that it wouldn't follow the cannula during its extraction from the patient's body. This causes the sampling operation to fail, and it is necessary to repeated the whole sampling procedure. The corresponding troubles and risks for the patient are therefore evident.

Moreover, the swinging movements of the cannula, which are necessary for obtaining the sample separation, create a further trauma as well as some micro-fractures in the bone and osteo-medullary patient's tissue, thus improving its sufferance and retarding the heal time.

A significant improvement in the above cited instrument is known from Italian patent N. 1.261.099. The biopsy instrument disclosed therein comprises, in addition to the aforesaid instrument structure, a cylindrical inner needle, which can be inserted into the cannula once the stylet has been pulled out and the frustule has been enclosed in the cannula.

This inner needle is provided, at its distal end, with a thin, approximately half-cylindrical lamina, which continues the shape of the same inner needle for a pre-defined angular portion thereof. It is inserted into the cannula until the lamina is interposed between the inner wall of the same cannula and the frustule, thus pressing against this latter. The tapered distal end of the cannula helps the lamina operation.

Further improvements of the above biopsy instrument are known. By example, the one described in the Italian patent N. 1.285.707, in the name of the same Applicant, comprises a hollow, cylindrical body, which is provided of a pair of approximately half-cylindrical laminas at its distal end. In fact, said laminas are the continuation of counter-faced sides of pre-defined angular of the cylindrical body. When this latter is pushed deeply inside the cannula, the distal portion of both laminas protrudes beyond the cannula distal end, converging one toward the other because of the tapering of the same cannula. Thus the converging action of the laminas separate the frustule end from the surrounding tissue, and also hold the same frustule while the cannula is being extracted from the patient's body. In this way the fail probabilities in the sampling operation are reduced and the frustule size is bigger, for a given depth of penetration. By contrary, sometimes a certain frustule squeezing can be noted, which could make the subsequent analysis somewhat difficult.

Several other kinds of biopsy instruments for taking bone or osteo-medullary biopsies are known. Nevertheless, all of them suffer of at least one of the drawbacks described above.

The main object of the present invention is to provide an instrument for making bone or osteo-medullary biopsies capable to achieve a good separation of the biopsic sample from the surrounding tissue, together with a certain sample retrieval, without affecting the sample itself and keeping it in an optimal state for the subsequent analysis.

A further object of the invention is to allow the use of a very thin cannula, compared with other known art biopsy instruments, for obtaining a frustule with a given diameter. Otherwise, with a cannula diameter equal to that of known art instruments, a frustule with a bigger diameter is obtained, thus allowing more reliable analysis.

Another object is to provide a biopsy instrument having a simpler structure and a lower cost than the prior art instruments, and which is also easy to use.

The aforesaid objects are fully achieved by an improved needle instrument for taking bone or osteo-medullary biopsic samples according to the contents of Claim 1.

The characteristic features of the present invention are highlighted in the following, with reference to the enclosed drawings, wherein:
- figure 1 shows a schematic, side sectional view of a first embodiment of the biopsy instrument according to the present invention, having the stylet inserted into the cannula;
- figure 2 shows a schematic, at an enlarged scale, a side sectional view of the distal portion of the instrument of figure 1;
- figure 3 shows a view according to the section plane III-III of figure 2;
- figure 4 shows the distal portion of the above instrument, rotated by 90° with respect to the view of figure 2, in a second embodiment of the invention;
- figure 5 shows a side sectional view of the portion of figure 2, rotated by 90°, during the sampling operation;
- figure 6 shows a side sectional view of the same portion of figure 2, after the instrument has been extracted from the patient's body.

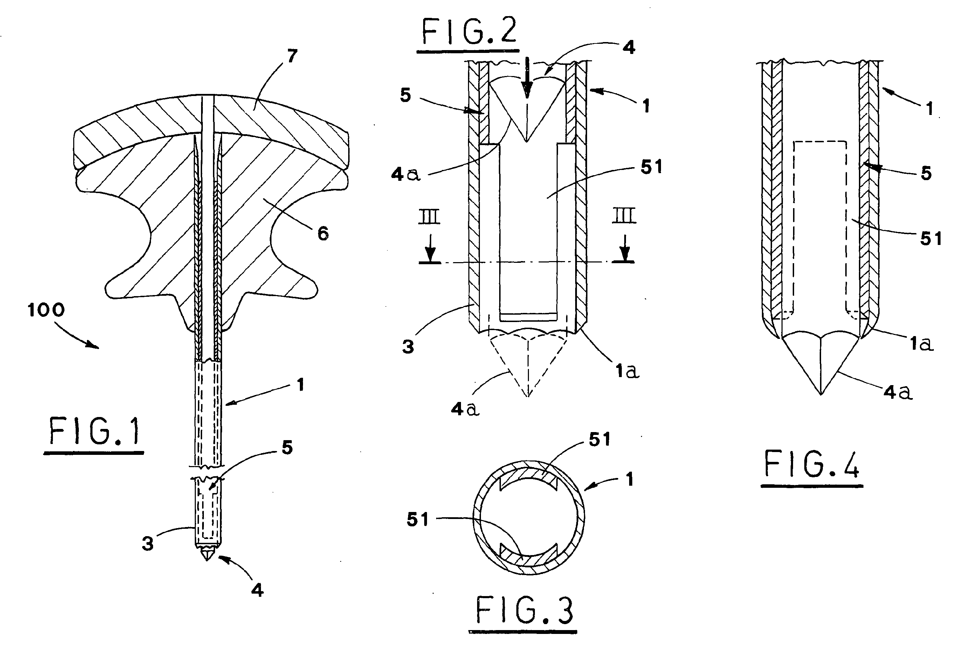

Referring now to figures 1 to 3, and to a first embodiment of the invention, numeral 100 indicates as a whole a needle instrument made according to the present invention. The instrument 100 can be used for manually performing a bone or osteo-medullary biopsy. It comprises a hollow cannula 1, having a substantially cylindrical shape. The distal end 1a of the aforesaid cannula 1 is sharpened and notched, in order to allow the same an easy penetration into a patient's osteo-medullary tissue.

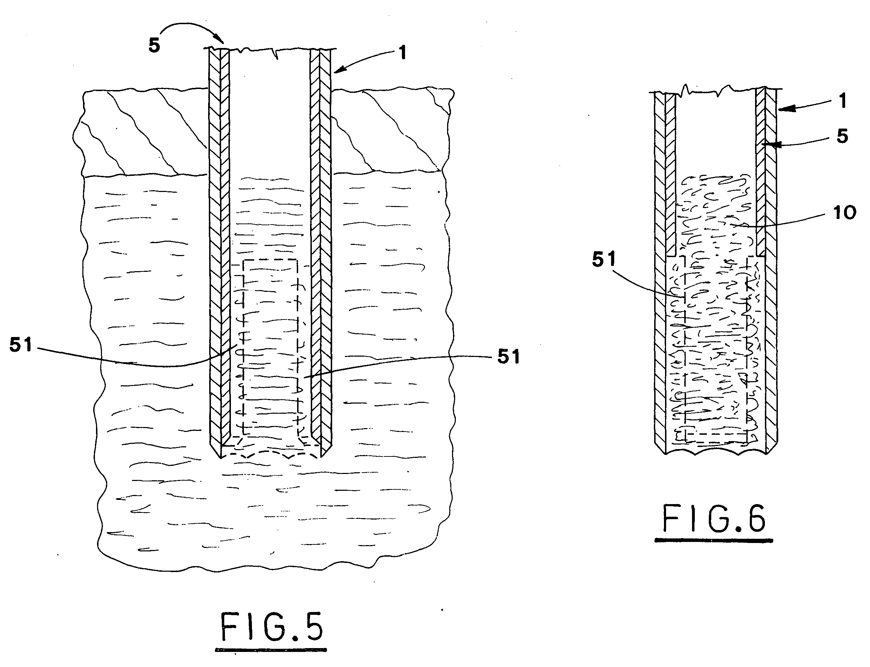

The cannula 1 is fit to receive a sample 10 of the above tissue, (see also figures 5 and 6) in its own hollow distal portion 3.

A handle 6 is fixed to the proximal end of cannula 1, for allowing an user to easily use the instrument 100 according to known operating modes.

A stylet 4 is also provided, which is fit to slide into the cannula 1 so as, once it has been fully inserted therein, its pointed distal end 4a protrudes out of the distal end of the same cannula 1. A stylet handle 7 is fixed to the proximal end of the stylet 4. The stylet handle 7 is fit to allow a user to insert or extract the same stylet 4 during the sampling operation, when a biopsic sample, or "frustule", is taken for subsequent analysis. The stylet handle 7 is preferably shaped to match the cannula handle 6, in order to be handled as a unique handle when the instrument 100 is being used.

A inner cannula 5 is fixed to the inner wall of the aforesaid cannula 1. The outer diameter of the inner cannula 5 is not greater than the inner diameter of cannula 1. The inner cannula 5 extends inside the cannula 1 until the above mentioned hollow distal portion 3 thereof. Preferably, the inner cannula 5 and the cannula 1 have the same length.

Although the inner cannula 5 can be conveniently soldered to the cannula 1, any other fixing techniques, as for example a gluing technique, can be used for achieving that scope.

The above cited stylet 4 has an outer diameter which is smaller than the inner diameter of the inner cannula 5, for allowing the same to be slidably inserted inside this latter.

A pair of protrusions 51 extends from the distal end of the inner cannula 5 and inside the hollow distal portion 3 of the cannula 1, towards the distal end 1a thereof. In fact, the protrusions 51 are the continuation of cylindrical sectors of the same inner cannula 5. Said protrusions define, inside the hollow distal portion 3, longitudinal zones of the same having a smaller inner diameter (those where the protrusions 51 lay) alternated to longitudinal zones having a bigger inner diameter.

The distal ends of the protrusions 51 are sharpened, for better brake up the osteo-medullary tissue during the operation of instrument 100.

As it can be seen in figure 2, since the cannula 1 is not tapered at its distal portion 1a, a small gap is present between the same distal portion and the stylet body when fully inserted into the cannula 1, shown with dashed lines. In any case said gap does not affect the sampling procedure.

In a second embodiment of the present invention, shown in figure 4, the distal end 1a of the cannula 1 is slightly tapered, so as to join up the pointed end 4a of the stylet 4. In this way the gap between cannula 1 and stylet 4 is removed. The sample diameter is then only slightly smaller, but this is not really significant.

Further structural variations, however falling into the scope of the present invention, comprise a different number of longitudinal protrusions 51. A single protrusion, as well as more than two, can be provided. Of course, when the number of protrusions 51 grows, the angular extension of each protrusion becomes smaller.

Moreover, cannula 1 and inner cannula 5 together with its longitudinal protrusions 51 can be made in a unique piece, by suitably working (e.g. milling) a single steel cannula having a suitable thickness.

In use, the needle instrument 100 made according to the present invention, having the inner cannula 5 permanently enclosed into the cannula 1 and the stylet 4 fully inserted, is pushed into the sampling site by perforating the patient's soft tissues and the bone layer and through the osteo-medullary tissue to be sampled.

The stylet 4 is then pulled up, and the cannula 1, together with the inner cannula 5, is pushed down in this latter tissue. An approximately cylindrical sample 10 of tissue is then enclosed into the distal portion 3a of cannula 1 (see figure 5). In the cannula areas when no protrusions 51 are present, the sample 10 diameter is larger, since it contacts the cannula 1 inner wall, than the sample diameter in the cannula areas where the protrusions 51 are present. Moreover, once it has been cut, the osteo-medullary sample naturally tends to expand. The sample 10 is the constrained to the instrument 100 with respect to rotations thereof.

The instrument 100 is then manually rotated several times, preferably alternating the rotation from clockwise to counterclockwise. As the sample 10 is constrained to rotate together with the cannulas 1,5, its distal end, which is still connected to the surrounding tissue, is subjected to torsional forces which cause its separation from said tissue. These simple rotations of the instrument 100 are not painful for the patient, nor increase the tissue damages significantly.

The cannulas are then extracted from the patient's body, together with the sample 10, which doesn't slide back from the cannula because it is fully separated from the osteo-medullary surrounding tissue. Then the sample 10 can be extracted from the distal end of cannula 1, without being damaged by this operation since the cannula distal end is not tapered. By contrary, in the biopsy instruments of known art, the sample has to be extracted from the cannula proximal end.

The present invention achieves some evident advantages over the prior art biopsy instruments. First, as the sample portions that lay between the longitudinal protrusions 51 constrain the sample with respect to the cannula rotations and, slightly, also with respect to a sliding movement, the sample separation and the subsequent sample recovery can occur without any further sufferance for the patient, and in a safe and reliable way.

Moreover, because no more devices must be inserted between the inner walls of the cannula 1 and the sample prior to recovery the same, a perfectly integral sample can be obtained.

A further advantage consists in that the sample can be recovered by extracting it from the cannula distal end, thus avoiding the stress that would occur to the sample during its transfer from the distal portion to the cannula proximal end.

The present invention has been described, with reference to the attached drawings, by way of an example, and thus without limiting its scope. It is therefore evident that all the modifications or variations suggested by practice or by the normal use of the invention, which are comprised into the following claims, will fall into the scope of the invention.

## Claims

1. Needle instrument for taking bone or osteo-medullary biopsies, comprising a hollow cannula (1), having its distal end (1a) sharpened for penetrate into a patient's osteo-medullary tissue, and fit to receive a sample (10) of said tissue in its own hollow distal portion (3), and also comprising a pointed stylet (4), slidably insertable into said cannula (1), said needle instrument (100) being **characterized in that** it moreover comprises an inner cannula (5) having a substantially cylindrical shape, fixed to the inner wall of said cannula (1) and extending for at least a part of said cannula (1) comprising said hollow distal portion (3), said inner cannula (5) being provided with at least one longitudinal protrusion (51), which is a continuation of a cylindrical sector of said inner cannula (5) and extending, inside said hollow distal portion (3), towards the distal end (1a) of said cannula (1).

2. Needle instrument according to Claim 1, **characterized in that** the distal end of said longitudinal protrusion (51) is sharpened.

3. Needle instrument according to Claim 1 or Claim 2, **characterized in that** it comprises a pair of counter-faced longitudinal protrusions (51).

4. Needle instrument according to Claim 1, **characterized in that** said distal end (1a) of cannula (1) is slightly tapered, for being connected with the corresponding pointed end (4a) of said stylet (4).

5. Needle instrument according to Claim 1, **characterized in that** said inner cannula (5) is fixed to said cannula (1) by soldering.

6. Needle instrument according to Claim 1, **characterized in that** said cannula (1) and inner cannula (5), together with its corresponding protrusions (51), are made of a single piece of metal.
